# EUROPEAN PATENT SPECIFICATION

(11) **EP 4 578 276 B1**
(45) Date of publication and mention of the grant of the patent: **07.10.2026**
(21) Application number: 23220889.2
(22) Date of filing: 31.12.2023
(51) Int. Cl.: A01K 29/00, A61B 5/103, A01K 1/06

(54) **DEVICE FOR DIAGNOSING THE CONDITION OF LAMNESS IN FOUR-LEGGED ANIMALS**
VORRICHTUNG ZUR DIAGNOSE DES LAHMHEITSSZUSTANDS BEI VIERBEINIGEN TIEREN
DISPOSITIF POUR DIAGNOSTIQUER LA CLAUDICATION CHEZ DES ANIMAUX QUADRUPÈDES

(43) Date of publication of application: 02.07.2025
(73) Proprietor: 7M Amdg Spolka z ograniczona odpowiedzialnoscia, 51-631 Wilczyce (PL)
(72) Inventor: ZDUNEK, Michal, 51-361 Wilczyce (PL); PIASECKI, Marek, 53-009 Wroc aw (PL); WEGLARSKI, Artur, 87-214 Pluznica (PL)
(74) Representative: JD&P Patent Attorneys Joanna Dargiewicz & Partners

(56) References cited:
- EP-B1- 2 097 002
- WO-A2-2020/167279
- CN-A- 103 960 157
- US-B2- 9 186 091

## Description

The present invention relates to a device for automating the diagnosis of lameness in cows and other four-legged animals with cow-like locomotion.

Lameness in cattle is a significant problem for farmers and ranchers, negatively affecting the health and performance of animals. Lameness is a condition, in which the animal experiences difficulty in walking or pain during movement. It is an issue, that requires attention due to its impact on the overall wellbeing and productivity of the herd.

Most importantly, lameness in cows leads to a decrease in milk yield, an increase in medical costs and a decrease in the value of the animals on the market. Animals with lameness may also show a reduced reproductive capacity, which in long term affects the profitability of the breeding.

There are several ways to detect lameness in cattle. The first is to observe the animals' behaviour. Farmers, by following the daily activities of the herd, can notice changes in gait, difficulties in movement or also changes in the animal's body position. In addition, farmers use monitoring technologies, such as cameras or motion sensors, to track the cattle's movements and detect possible health problems early.

European patent EP2097002 B1 discloses a device for capturing a gait pattern of a living being, comprising a tread plate and at least one sensor, associated with the tread plate or integrated into it, a sensor signal of the at least one sensor being supplied to an evaluation device.

Accurate diagnosis of lameness in cows requires the cooperation of a veterinarian, who can use various methods such as radiological examinations or assessment of gait biomechanics. It is important to identify and treat cases of lameness quickly, in order to minimise negative effects on animal health, and maintain high quality milk and meat production. Breeders are working to continuously improve husbandry practices and introduce innovative solutions to effectively manage lameness in cattle.

The object of the present invention was to develop a device for detecting and/or monitoring lameness in cows and other quadrupedal animals.

This objective has been achieved by the subject of the present invention, which is a device for diagnosing the condition of lameness in quadrupedal animals, in particular cows and other quadrupedal animals with cow-like locomotion, which comprises:
- a floor, in which at least four rectangular strain gauge platforms are installed in a checkerboard pattern, equipped with strain gauge sensors, enabling measurement of the pressure of the animal's legs placed on these platforms;
- a fence, adjacent directly to the outer edges of the strain gauge platforms, limiting the movement of the animal during the measurement;
- at least one stimulus generator, per strain gauge platform, initiating variations in the load distribution between individual legs;
- a system to record and display the measured load per individual strain gauge platforms.

Preferably, the device additionally comprises an input gate and an output gate, allowing the animal to enter the platform before the measurement, and to exit it after completion of the measurement, or one input-output gate, respectively, which constitutes an input gate and an output gate simultaneously.

Preferably, the device additionally has a data analysis system which, based on the data from the system collecting and presenting the measured pressures on the individual strain gauge platforms, determines the degree of lameness in the tested animal.

Preferably, the device allows reading of microchips in the standard ISO 11784 / 11785.

Preferably, the device is provided with at least one stimulus generator per strain gauge platform, in the form of an arm, touching the lower parts of the legs, at the level of the hooves, cloven hooves or fetlocks, forcing the limb to be lifted or moved.

Preferably, the device is provided with two or four stimulus generators per strain gauge platform.

Preferably, the device is provided with at least one stimulus generator, per strain gauge platform, in the form of lateral arms, movable planes/barriers, affecting the sides of the animal at the height of the scapula, ribs, flanks, forcing the entire body of the animal to tilt.

Preferably, at least one stimulus generator is a milking robot arm.

Preferably, the device is provided with at least one stimulus generator per strain gauge platform, in the form of a nozzle enabling the pulsed delivery of a stream of fluid, preferably water or compressed air, to selected areas of the animal's body.

Preferably, the stimulus generator administers a jet of fluid to the flanks in the groin area, at the hind legs, or in the scapula area, at the fore legs.

Preferably, the stimulus generators feeding the fluid streams are arranged at different heights of the fence.

Preferably, the device is an independent diagnostic device.

Preferably, the device is positioned in a communication path, by which the animals move to/from milking or to other elements of the farm, such as to watering or feeding stations.

Preferably, the device is connected to an automatic milking station.

Preferably, the device is connected to an automatic feed station.

The device (platform) according to the present invention comprises the following elements:
(a) a floor, in which are mounted rectangular measuring segments SP, in a checkerboard arrangement, containing strain gauge sensors to measure the pressure of the animal's legs placed on these segments. The device uses between 4 and 8 SP segments (at least one separate segment for each leg). The number of segments depends, among other things, on the size of the cow and where the device is installed.
(b) A measuring cage, a fence, which restricts significant movement of the animal during measurement, such that at all times during measurement each of the animal's legs is either placed on one of the SP segments or is in the air (raised) above the measuring segment. The design of the cage limits the possibility of legs being placed outside the SP segments. The cage may also have automatic or manual closing gates (two separate: entrance and exit, or one: entrance and exit), allowing the animal to enter the platform before measurement and to leave it after measurement.
   a) Effectors of the artificial stimulus system, initiating changes in the animal's position (changes in the distribution of weight between the legs). This is additional, optional equipment, fixed in various configurations to the platform structure, in the form of four different types of affects:
      **stimulus b. A)** in the form of four or two mobile arms, touching the lower parts of the legs (at the level of: hooves, cloven hooves or fetlocks), forcing the limb to be lifted or moved, in the automated variant such an arm could be a milking robot arm, that can be slid under the cow's body, between the four legs, affecting the lower parts of the legs, for example by touching the legs from the inside towards the outside, which can be even more effective, because the inner part of the leg is less exposed to abrasion and injury, so it should be more sensitive
      **stimulus b.B)** of lateral arms, forcing a change of position - moving planes/barriers acting on the sides of the animal (at the level of the scapula - ribs - flanks), forcing a tilt of the whole body of the animal and consequently a change of weight distribution on the legs
      **stimulus b.C)** of a nozzle, allowing the pulsed application of a jet of liquid (e.g. water) or compressed air, to selected areas of the animal's body (e.g. to the so-called flanks in the groin area, near the hind legs, or to the scapula area, near the fore legs),
      **stimulus b.D)** the last, specific type of stimulus is the construction of the measuring platform itself, acting as a fence restricting the cow's natural behavioural freedom and mobility. During measurements lasting longer, e.g. from 3 to 30 minutes, there will be a natural need for individual leg muscles to rest and fatigue will set in for an animal standing in the same position for a long time. Such a stimulus will also cause the animal to adopt different positions, changing the distribution of weight between the different legs and raising and lowering them.

A characteristic feature of the lameness measurement method described here, is that an 'active' measurement is made, in which the relationships between selected elements of the locomotor system of the standing cow are investigated, in particular the changes in the distribution of the total body weight between the different legs, as the animal assumes different positions (body configurations), in response to artificially (externally) administered stimuli. The general concept of such an examination is similar to active diagnostic methods used in medicine, such as the classic human neurological examination, which involves applying a hammer to the patellar ligament of the knee and observing the response in the form of quadriceps contraction.

This is an important difference to methods of diagnosing cow lameness, described in the scientific literature (e.g. on the basis of movement recordings from video cameras) or strain gauge measurements (e.g. the commercial StepMetrix device from BowMatic), which are "passive" in nature. That is, they are based on measurements of the natural - undisturbed - behaviour of the passing animal. In these methods, the introduction of external influences on the cow's behaviour is regarded as a source of errors, that prevents correct measurement.

The device according to the present invention can be used in several different configurations with typical equipment and work organisation in cow husbandry:
1) In the simplest variant, which does not require significant organisational changes in the operation of the kennel, the device can operate as an independent diagnostic device, to which animals are brought by the kennel staff, and some manipulation activities (e.g. opening/closing gates) can be performed manually. In this variant, the timing of the measurement is not critical, so it can take a few or even several minutes. As a result, in addition to measurements with b.A/B/C stimuli, it is also possible to perform a measurement with a b.D stimulus in the form of an "extended test time".
2) In a more automated version, the device can be placed in the communication line, by which the animals move to/from milking (similar to the StepMetrix system), or to other elements of the kennel, e.g. watering or feeding stations. In this version, it is necessary to additionally use a system of automatic opening and closing of the platform gates, and control of bypassing movement of animals, that are not intended for testing. In this variant, the duration of the survey will be critical, and should be as short as possible, preferably less than one minute, so as not to block the movement of other animals. This variant necessarily requires the use of additional artificially administered stimuli (b.A/B/C), so that every few seconds the animal makes a change in locomotor configuration (lifts the stimulated legs and changes the distribution of its weight over the remaining legs).
3) It is also possible to combine the device according to the present invention with an automatic milking station. In this case, the typical residence time of the cow in the milking stall (of the order of 3-6 minutes = udder cleaning time + attachment time of the milking equipment + milking time) will be sufficient to collect the necessary observations. The manipulator of the milking robot touching different parts of the cow's body (during cleaning, attachment and milking) will act as an effector of the b.A. system.
4) It is also possible to combine the measuring device with an automatic feed station. A longer cow stay at the feed station (of the order of a few-several minutes) will even allow the measurement to be divided into two phases: F1) a longer phase of passive measurement during the feeding time (in order not to interfere with feed intake, only stimulus type b.D is used in this phase) and a shorter phase F2) at the end of the feeding time, when the use of stimuli type b.A/B/C will mobilise the animal to finish feed intake and leave the feed station, and at the same time will make it possible to analyse changes in the locomotor state during this process.

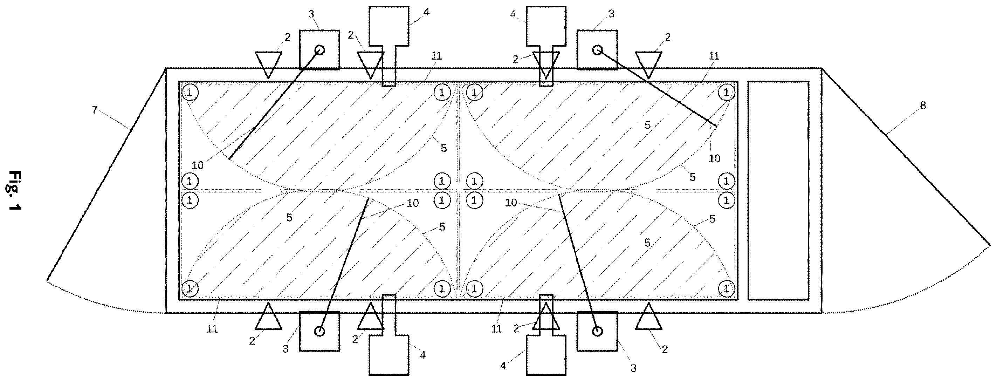

The object of the present invention is illustrated in the figures of the drawing, where
Fig. 1 shows a device according to the present invention in plan view;

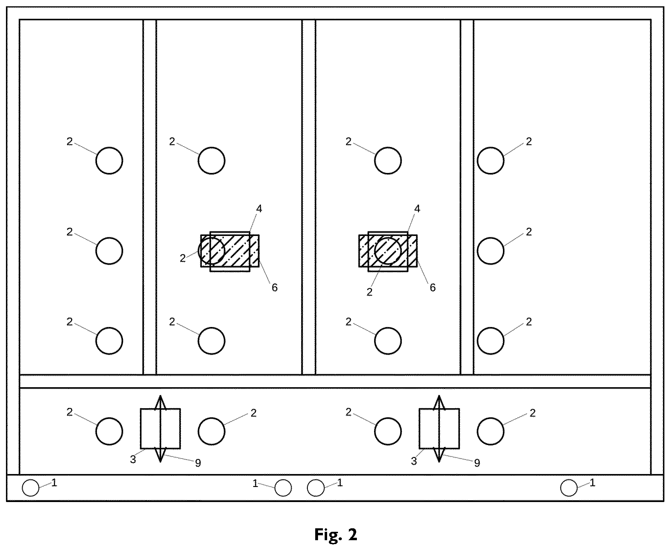

Fig. 2 shows a device according to the present invention in side view;

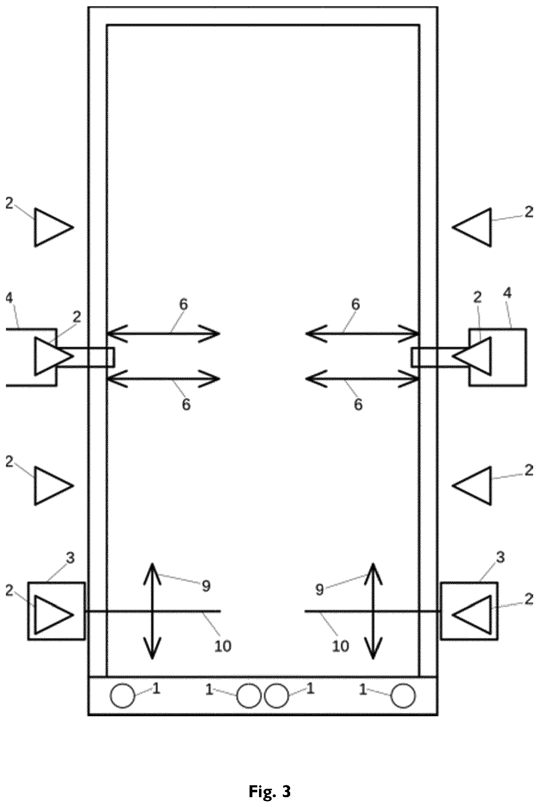

Fig. 3 shows a device according to the present invention in front view.

### Legend

1 - Strain gauges and their exemplary installation
2 - Fluidic stimulus generators
3 - Lower stimulus generators
4 - Upper stimulus generators
5 - Operating range of lower generators
6 - Operating range of upper generators
7 - Input gate
8 - Output gate
9 - Height adjustment range of lower stimulus generator
10 - Lower stimulus arm
11 - Single strain gauge platform

## Claims

1. A device for diagnosing the condition of lameness in quadrupedal animals, in particular cows and other quadrupedal animals with cow-like locomotion, **characterised in that** it comprises:
- a floor, in which at least four rectangular strain gauge platforms (11) are installed in a checkerboard pattern, equipped with strain gauge sensors (1), enabling measurement of the pressure of the animal's legs placed on these platforms;
- a fence, adjacent directly to the outer edges of the strain gauge platforms (11), limiting the movement of the animal during the measurement;
- at least one stimulus generator (2), (3) and/or (4) per strain gauge platform (11), initiating variations in the load distribution between individual legs, wherein the stimulus generator (2) is in the form of a nozzle enabling the pulsed delivery of a stream of fluid, preferably water or compressed air, to selected areas of the animal's body, the stimulus generator (3) is in the form of an arm touching the lower parts of the legs, at the level of the hooves, cloven hooves or fetlocks, forcing the limb to be lifted or moved, and the stimulus generator (4) is in the form of lateral arms, movable planes/barriers, affecting the sides of the animal at the height of the scapula, ribs, flanks, forcing the entire body of the animal to tilt;
- a system to record and display the measured load per individual strain gauge platforms (11).

2. The device according to claim 1, **characterised in that** it additionally comprises an input gate (7) and an output gate (8), allowing the animal to enter the platform before the measurement, and to exit it after completion of the measurement, or one input-output gate, respectively, which constitutes an input gate (7) and an output gate (8) simultaneously.

3. The device according to claim 1 or 2, **characterised in that** it additionally has a data analysis system which, based on the data from the system collecting and presenting the measured pressures on the individual strain gauge platforms (11), determines the degree of lameness in the tested animal.

4. The device according to claim 1 or 2 or 3, **characterised in that** it allows reading of microchips in the standard ISO 11784 / 11785.

5. The device according to any of the claims 1-4, **characterized in that** it is provided with two or four stimulus generators (3) per strain gauge platform (11).

6. The device according to any of the claims 1-5, **characterized in that** at least one stimulus generator, (3) or (4), is a milking robot arm.

7. The device according to any of the claims 1-6, **characterized in that** the stimulus generator (2) administers a jet of fluid to the flanks in the groin area, at the hind legs, or in the scapula area, at the fore legs.

8. The device according to any of the claims 1-7, **characterized in that** the stimulus generators (2) are arranged at different heights of the fence.

9. The device according to any of the claims 1-8, **characterized in that** it is an independent diagnostic device.

10. The device according to any of the claims 1-9, **characterized in that** it is positioned in a communication path, by which the animals move to/from milking or to other elements of the kennel, such as to watering or feeding stations.

11. The device according to any of the claims 1-10, **characterized in that** it is connected to an automatic milking station.

12. The device according to any of the claims 1-10, **characterized in that** it is connected to an automatic feed station.

## Patentansprüche

1. Vorrichtung zur Diagnose von Lahmheit bei vierfüßigen Tieren, insbesondere bei Kühen und anderen vierfüßigen Tieren mit kuhähnlicher Fortbewegung, **dadurch gekennzeichnet, dass** sie umfasst:
- einen Boden, in dem mindestens vier rechteckige Dehnungsmessstreifen-Plattformen (11) in einem Schachbrettmuster angeordnet sind, die mit Dehnungsmessstreifen-Sensoren (1) ausgestattet sind und die Messung des Drucks der auf diesen Plattformen aufgesetzten Beine des Tieres ermöglichen;
- eine Umzäunung, die direkt an die Außenkanten der Dehnungsmessstreifen-Plattformen (11) angrenzt und die Bewegung des Tieres während der Messung begrenzt;
- mindestens einen Reizgenerator (2), (3) und/oder (4) pro Dehnungsmessplattform (11), der Schwankungen in der Lastverteilung zwischen den einzelnen Beinen auslöst, wobei der Reizgenerator (2) die Form einer Düse hat, die die gepulste Abgabe eines Fluidstroms, vorzugsweise Wasser oder Druckluft, an ausgewählte Bereiche des Tierkörpers ermöglicht, der Reizgenerator (3) die Form eines Arms hat, der die unteren Teile der Beine berührt, auf Höhe der Hufe, Spaltenhufe oder Fesseln, wodurch die Gliedmaße zum Anheben oder Bewegen gezwungen wird, und der Reizgenerator (4) in Form von seitlichen Armen oder beweglichen Ebenen/Barrieren vorliegt, die auf die Seiten des Tieres in Höhe des Schulterblatts, der Rippen und der Flanken einwirken und den gesamten Körper des Tieres zum Kippen zwingen;
- ein System zur Erfassung und Anzeige der gemessenen Belastung pro einzelner Dehnungsmessstreifen-Plattform (11).

2. Vorrichtung nach Anspruch 1, **dadurch gekennzeichnet, dass** sie zusätzlich ein Eingangs- (7) und ein Ausgangstor (8) umfasst, die es dem Tier ermöglichen, die Plattform vor der Messung zu betreten und sie nach Abschluss der Messung zu verlassen, oder jeweils ein Ein- und Ausgangstor, das gleichzeitig als Eingangs- (7) und Ausgangstor (8) dient.

3. Vorrichtung nach Anspruch 1 oder 2, **dadurch gekennzeichnet, dass** sie zusätzlich ein Datenanalysesystem aufweist, das auf der Grundlage der Daten aus dem System zur Erfassung und Darstellung der gemessenen Drücke auf den einzelnen Dehnungsmessstreifen-Plattformen (11) den Grad der Lahmheit des untersuchten Tieres bestimmt.

4. Vorrichtung nach Anspruch 1 oder 2 oder 3, **dadurch gekennzeichnet, dass** sie das Auslesen von Mikrochips gemäß der Norm ISO 11784/11785 ermöglicht.

5. Vorrichtung nach einem der Ansprüche 1 bis 4, **dadurch gekennzeichnet, dass** sie mit zwei oder vier Reizgeneratoren (3) pro Dehnungsmessstreifen-Plattform (11) ausgestattet ist.

6. Vorrichtung nach einem der Ansprüche 1 bis 5, **dadurch gekennzeichnet, dass** mindestens ein Reizgenerator (3) oder (4) ein Melkroboterarm ist.

7. Vorrichtung nach einem der Ansprüche 1 bis 6, **dadurch gekennzeichnet, dass** der Reizgenerator (2) einen Flüssigkeitsstrahl auf die Flanken im Leistenbereich, an den Hinterbeinen oder im Schulterblattbereich an den Vorderbeinen richtet.

8. Vorrichtung nach einem der Ansprüche 1 bis 7, **dadurch gekennzeichnet, dass** die Reizgeneratoren (2) in unterschiedlichen Höhen am Zaun angeordnet sind.

9. Vorrichtung nach einem der Ansprüche 1 bis 8, **dadurch gekennzeichnet, dass** es sich um eine eigenständige Diagnosevorrichtung handelt.

10. Vorrichtung nach einem der Ansprüche 1 bis 9, **dadurch gekennzeichnet, dass** sie in einem Laufweg positioniert ist, über den sich die Tiere zum/vom Melken oder zu anderen Elementen des Stalls, wie beispielsweise zu Tränk- oder Futterstellen, bewegen.

11. Vorrichtung nach einem der Ansprüche 1 bis 10, **dadurch gekennzeichnet, dass** sie mit einer automatischen Melkstation verbunden ist.

12. Vorrichtung nach einem der Ansprüche 1 bis 10, **dadurch gekennzeichnet, dass** sie mit einer automatischen Futterstation verbunden ist.

## Revendications

1. Dispositif destiné à diagnostiquer l'état de boiterie chez les animaux quadrupèdes, en particulier les vaches et autres animaux quadrupèdes ayant une locomotion similaire à celle des vaches, **caractérisé en ce qu'**il comprend :
- un plancher, dans lequel au moins quatre plateformes rectangulaires à jauges de contrainte (11) sont installées selon un motif en damier, équipées de capteurs à jauges de contrainte (1), permettant de mesurer la pression exercée par les pattes de l'animal posées sur ces plateformes ;
- une barrière, adjacente directement aux bords extérieurs des plateformes à jauges de contrainte (11), limitant les mouvements de l'animal pendant la mesure ;
- au moins un générateur de stimuli (2), (3) et/ou (4) par plate-forme à jauges de contrainte (11), provoquant des variations dans la répartition de la charge entre les pattes individuelles, le générateur de stimuli (2) se présentant sous la forme d'une buse permettant l'émission pulsée d'un jet de fluide, de préférence de l'eau ou de l'air comprimé, vers des zones sélectionnées du corps de l'animal, le générateur de stimuli (3) se présentant sous la forme d'un bras touchant les parties inférieures des pattes, au niveau des sabots, des sabots fendus ou des boulets, forçant le membre à être soulevé ou déplacé, et le générateur de stimuli (4) se présente sous la forme de bras latéraux, de plans ou de barrières mobiles, agissant sur les flancs de l'animal à la hauteur de l'omoplate, des côtes et des flancs, forçant l'ensemble du corps de l'animal à s'incliner ;
- un système d'enregistrement et d'affichage de la charge mesurée par chaque plate-forme à jauges de contrainte (11).

2. Dispositif selon la revendication 1, **caractérisé en ce qu'**il comprend en outre une porte d'entrée (7) et une porte de sortie (8), permettant à l'animal d'entrer sur la plate-forme avant la mesure et d'en sortir une fois la mesure terminée, ou une porte d'entrée-sortie unique, constituant à la fois une porte d'entrée (7) et une porte de sortie (8).

3. Dispositif selon la revendication 1 ou 2, **caractérisé en ce qu'**il comporte en outre un système d'analyse des données qui, sur la base des données provenant du système de collecte et de présentation des pressions mesurées sur les différentes plateformes à jauges de contrainte (11), détermine le degré de boiterie chez l'animal testé.

4. Dispositif selon la revendication 1, 2 ou 3, **caractérisé en ce qu'**il permet la lecture de puces électroniques conformes à la norme ISO 11784 / 11785.

5. Dispositif selon l'une quelconque des revendications 1 à 4, **caractérisé en ce qu'**il est muni de deux ou quatre générateurs de stimuli (3) par plate-forme à jauges de contrainte (11).

6. Dispositif selon l'une quelconque des revendications 1 à 5, **caractérisé en ce qu'**au moins un générateur de stimuli (3) ou (4) est un bras de robot de traite.

7. Dispositif selon l'une quelconque des revendications 1 à 6, **caractérisé en ce que** le générateur de stimuli (2) administre un jet de fluide sur les flancs au niveau de l'aine, au niveau des pattes arrière, ou au niveau des omoplates, au niveau des pattes avant.

8. Dispositif selon l'une quelconque des revendications 1 à 7, **caractérisé en ce que** les générateurs de stimuli (2) sont disposés à différentes hauteurs de la barrière.

9. Dispositif selon l'une quelconque des revendications 1 à 8, **caractérisé en ce qu'**il s'agit d'un dispositif de diagnostic autonome.

10. Dispositif selon l'une quelconque des revendications 1 à 9, **caractérisé en ce qu'**il est positionné sur un parcours emprunté par les animaux pour se rendre à la traite ou en revenir, ou pour se rendre à d'autres éléments de l'étable, tels que des points d'abreuvement ou d'alimentation.

11. Dispositif selon l'une quelconque des revendications 1 à 10, **caractérisé en ce qu'**il est relié à une station de traite automatique.

12. Dispositif selon l'une quelconque des revendications 1 à 10, **caractérisé en ce qu'**il est relié à une station d'alimentation automatique.
